# EUROPEAN PATENT SPECIFICATION

(11) **EP 3 472 615 B1**
(45) Date of publication and mention of the grant of the patent: **19.08.2020**
(21) Application number: 17734853.9
(22) Date of filing: 14.06.2017
(51) Int. Cl.: G01N 33/50, G01N 33/68

(54) **MARKERS AND THEIR RATIO TO DETERMINE THE RISK FOR EARLY-ONSET PREECLAMPSIA**
MARKER UND DEREN VERHÄLTNIS ZUR FESTSTELLUNG DES RISIKOS VON FRÜH EINSETZENDER PRÄEKLAMPSIE
MARQUEURS ET LEUR RAPPORT POUR DÉTERMINER LE RISQUE DE PRÉ-ÉCLAMPSIE PRÉCOCE

(30) Priority: 15.06.2016 NL 2016967
(43) Date of publication of application: 24.04.2019
(73) Proprietor: IQ Products B.V., 9727 DL Groningen (NL)
(72) Inventor: SCHUITEMAKER, Joost Henric Nicolaas, 9727 DL Groningen (NL)
(74) Representative: EDP Patent Attorneys B.V.
(86) International application number: PCT/NL2017/050392
(87) International publication number: WO 2017/217844

(56) References cited:
- WO-A1-2009/066821
- WO-A2-2004/003172
- WO-A2-2004/021978
- US-A1- 2006 154 316
- SARAH CROSS ET AL: "Endocan (ESM-1): a novel soluble endothelial cell injury marker in preeclampsia (PE) and intrauterine growth restriction (IUGR)", 33RD ANNUAL MEETING/PREGNANCY MEETING OF THE SOCIETY-FOR-MATERNAL-FETAL-MEDICINE (SMFM); SAN FRANCISCO, CA, USA, vol. 208, 20 December 2012 (2012-12-20), page S276, XP055121155, DOI: 1
- OLIVER BLEIZIFFER ET AL: "Guanylate-binding protein 1 expression from embryonal endothelial progenitor cells reduces blood vessel density and cellular apoptosis in an axially vascularised tissue-engineered construct", BMC BIOTECHNOLOGY, BIOMED CENTRAL LTD. LONDON, GB, vol. 12, no. 1, 6 December 2012 (2012-12-06), page 94, XP021141550, ISSN: 1472-6750, DOI: 10.1186/1472-6750-12-94 cited in the application
- IBRAHIM SHERRINE A ET AL: "Inflammatory gene networks in term human decidual cells define a potential signature for cytokine-mediated parturition", AMERICAN JOURNAL OF OBSTETRICS & GYNECOLOGY, MOSBY, ST LOUIS, MO, US, vol. 214, no. 2, 5 September 2015 (2015-09-05), XP029406645, ISSN: 0002-9378, DOI: 10.1016/J.AJOG.2015.08.075

## Description

### FIELD OF THE INVENTION

The invention relates to the detection of preeclampsia.

### BACKGROUND OF THE INVENTION

Methods for diagnosing preeclampsia are known in the art. WO2013/071703 for instance describes a means for rapidly detecting Adipsin for diagnosing preeclampsia. This comprises a water-adsorbing pad, a nitrocellulose membrane, a gold labeling pad and a sampling pad, all of which are successively joined from the top down and fixed in the base plate. The gold-labeling pad is partly overlapped by the sampling pad. A detecting line coated by rabbit anti-human Adipsin polyclonal antibody, or goat anti-human Adipsin polyclonal antibody or mouse anti-human Adipsin polyclonal antibody and a controlling line coated by goat anti-mouse IgG polyclonal antibody are provided on the nitrocellulose membrane. The detecting line is located downstream and spaced from the controlling line. The gold-labeling pad is made of water adsorbing material, and coated by gold labeled mouse anti-human Adispin monoclonal antibody. A test kit for rapidly detecting Adipsin for diagnosing preeclampsia comprises an outer house and the said means therein. The method for preparing the said detecting means comprise: 1) coating of the detecting line and the controlling line; 2) preparing the gold labeling pad; 3) combination.

Further WO2014/001244 describes diagnostic assays for prenatal diagnosis of preeclampsia. Amongst others it describes a method for diagnosing whether a pregnant subject is not at risk for preeclampsia within a short window of time comprising a) determining the amount of at least one angiogenesis biomarker selected from the group consisting of sFlt-1, Endoglin and P1GF in a sample of said subject, and b) comparing the amount with a reference, whereby a subject being not at risk for developing preeclampsia within a short period of time is diagnosed if the amount is identical or decreased compared to the reference in the cases of sFlt-1 and Endoglin and identical or increased in the case of P1GF, wherein said reference allows for making the diagnosis with a negative predictive value of at least about 98%. Further contemplates are devices and kits for carrying out said method.

WO2014/135488 describes an in vitro method for identifying a pregnancy related syndrome selected from the group consisting of pre-eclampsia, eclampsia, Hemolysis Elevated Liver enzymes and Low Platelets (HELLP) and intra-uterine growth restriction (IUGR), the method including (i) measuring the amount of ESM-1 in a biological fluid sample from a pregnant subject, wherein the pregnant subject is between week 1 to 20 of gestation; (ii) comparing said amount of ESM-1 to a reference value, and (iii) identifying the subject as being likely to have or develop the pregnancy related syndrome based on a comparison of the amount of ESM-1 to the reference value. A device and a kit for identifying such a pregnancy related syndrome are also claimed.

US2014/0243212 describes methods for diagnosing pregnancy-associated disorders, determining allelic ratios, determining maternal or fetal contributions to circulating transcripts, and/or identifying maternal or fetal markers using a sample from a pregnant female subject. Also provided is use of a gene for diagnosing a pregnancy-associated disorder in a pregnant female subject.

The paper "Early Pregnancy Biomarkers in Pre-Eclampsia: A Systematic Review and Meta-Analysis", by Wu et al., Int. Journal of Molecular Sciences, (2015) Vol. 16, p. 23035-23056, describes that Pre-eclampsia (PE) complicates 2%-8% of all pregnancies and is an important cause of perinatal morbidity and mortality worldwide. In order to reduce these complications and to develop possible treatment modalities, it is important to identify women at risk of developing PE. The use of biomarkers in early pregnancy would allow appropriate stratification into high and low risk pregnancies for the purpose of defining surveillance in pregnancy and to administer interventions. We used formal methods for a systematic review and meta-analyses to assess the accuracy of all biomarkers that have been evaluated so far during the first and early second trimester of pregnancy to predict PE. We found low predictive values using individual biomarkers which included a disintegrin and metalloprotease 12 (ADAM-12), inhibin-A, pregnancy associated plasma protein A (PAPP-A), placental growth factor (PIGF) and placental protein 13 (PP-13). The pooled sensitivity of all single biomarkers was 0.40 (95% CI 0.39-0.41) at a false positive rate of 10%. The area under the Summary of Receiver Operating Characteristics Curve (SROC) was 0.786 (SE 0.02). When a combination model was used, the predictive value improved to an area under the SROC of 0.893 (SE 0.03). In conclusion, although there are multiple potential biomarkers for PE their efficacy has been inconsistent and comparisons are difficult because of heterogeneity between different studies. Therefore, there is an urgent need for high quality, large-scale multicentre research in biomarkers for PE so that the best predictive marker(s) can be identified in order to improve the management of women destined to develop PE.

US2006/0154316 describes a method for the identification and/or quantification of GBP-1 or fragments of this protein in the culture supernatant of a tissue sample, a body fluid sample or a sample from a cell culture supernatant. The publication "Endocan (ESM-1): a novel soluble endothelial cell injury marker in preeclampsia (PE) and intrauterine growth restriction (IUGR)" by S. Cross et al., 33rd annual meeting/pregnancy meeting of the society for maternal fetal medicine; American Journal of Obstetrics & Gynecology (2012) vol. 208, page S276 describes an vitro method for identifying early-onset severe preeclampsia (sPE) from a pregnant subject based on the amount of ESM-1 was measured in serum samples.

WO2004/0021978 describes antisense compounds, compositions, and methods provided for modulating the expression of Endothelial Specific Molecule-1 (ESM-1). The compositions comprise antisense compounds, particularly antisense oligonucleotides, targeted to nucleic acids encoding ESM-1. Methods of using these compounds for modulation of ESM-1 expression and for treatment of diseases associated with expression of ESM-1 are provided.

WO2004/003172A2 describes a nucleic acid and its encoded polypeptide ESM-1, whose expression is modulated in angiogenesis and oncogenesis; antibodies having specificity for said polypeptide; antisense molecules; and methods useful for treating or modulating angiogenesis in mammals in need of such biological effect.

### SUMMARY OF THE INVENTION

Preeclampsia is a syndrome which is characterized by high blood pressure (>140/90) and significant concentrations of protein in the urine (>300 mg/24 hrs urine) predominantly during the last weeks of pregnancy. Two forms are clinically recognized; the early-onset preeclampsia, which appears before the 34th week gestational age (GA), and the late-onset preeclampsia, which appears after the 34th week of GA. If left untreated the condition might result in, for both mother and child, long term effects and even a life-threatening situation. And although some women benefit from a low dose Aspirin supplementation or can be stabilized by intravenous magnesium sulfate, the best treatments for preeclampsia or advancing preeclampsia are abortion or delivery. This makes monitoring for and treatment of the early-onset preeclampsia even more important since this increases the chances for mother and child, simply because of the fact that delivery should be delayed as long as possible.

Currently, a lot of research is focused on discovering the underlying cause. These efforts have led to the discovery of several factors that play a role in the maternal immune system and, moreover, these findings point towards the importance of effective gestational immune tolerance in preeclampsia.

The current understanding of the syndrome is that it is a two-stage process. The initial lack of gestational immune tolerance of the placental cytotrophoblasts may lead to inadequately remodeled spiral arteries and a shallow implantation, which in turn leads to downstream hypoxia. This hypoxia of the placenta seems to be an important factor, since it is followed by the release of soluble factors in the maternal circulation. Some soluble factors, like soluble Fms-like tyrosine kinase-1 (sFlt-1 or sVEGFR-1) and soluble Endoglin (sEng), have been described as predictive markers which are differentially expressed in preeclamptic pregnancies after week 20 of gestation compared to healthy pregnancies. These factors are currently used for screening during pregnancy and their concentrations give an indication of the severity of the disease.

Guanylate binding protein-1 (GBP-1) is a large GTPase (guanosine triphosphate hydrolase) and is produced in response to IFNgamma and confers resistance to viral infections. Additionally, GBP-1 has been identified as an intracellular inhibitor of endothelial cell proliferation, invasion, and migration and therefore has a role in angiogenesis. The expression of the protein by endothelial cells has a negative effect on the differentiation of progenitor cells and their capacity to migrate and for this reason can be considered anti-angiogenic (Bleiziffer et al. BMC Biotechnology 2012, 12:94). Although GBP-1 is acting intracellular, the protein is secreted under pathological conditions. Remarkably, secretion of GBP-1 by endothelial cells is signal sequence independently and as a consequence it can be measured in plasma and serum of mammals (Hammon M, et al. J. Cell. Mol. Med. Vol. 15, No. 7, 2011 pp. 1582-1592).

In 1996 a new marker for endothelial cells was discovered by Lassalle et al. (J. Biol. Chem. 1996, 271:20458-20464). This proteoglycan of 50 kD was called endothelial cell-specific molecule 1 (ESM-1) and is also known as Endocan. The expression and secretion of ESM-1 is upregulated by pro-inflammatory molecules, such as TNF alpha, and in the presence of pro-angiogenic factors such as VEGF. The molecule has been described to be upregulated in inflammatory conditions like sepsis, but also in cancer. The fact that ESM-1 is involved in blood vessel growth and has prognostic value in relation to preeclampsia has been shown in PCT application PCT/EP2014/054053.

Since GBP-1 and ESM-1 both play a role in the regulation of angiogenesis and they respond to inflammatory factors, like IFNgamma, we hypothesized that the expression of both proteins during pregnancy would be different between the non pregnant (n=25), healthy pregnant (n=32) and the two forms, early (n=52) and late (n=13) onset, of preeclampsia pregnancies. Therefore, in a first experiment 117 plasma samples were obtained in the last trimester from pregnant women suffering from early and late-onset preeclampsia as well as healthy pregnancies as a control. The samples and controls were matched on the level of gestational age. The concentrations of GBP-1 and ESM-1 were determined by ELISA.

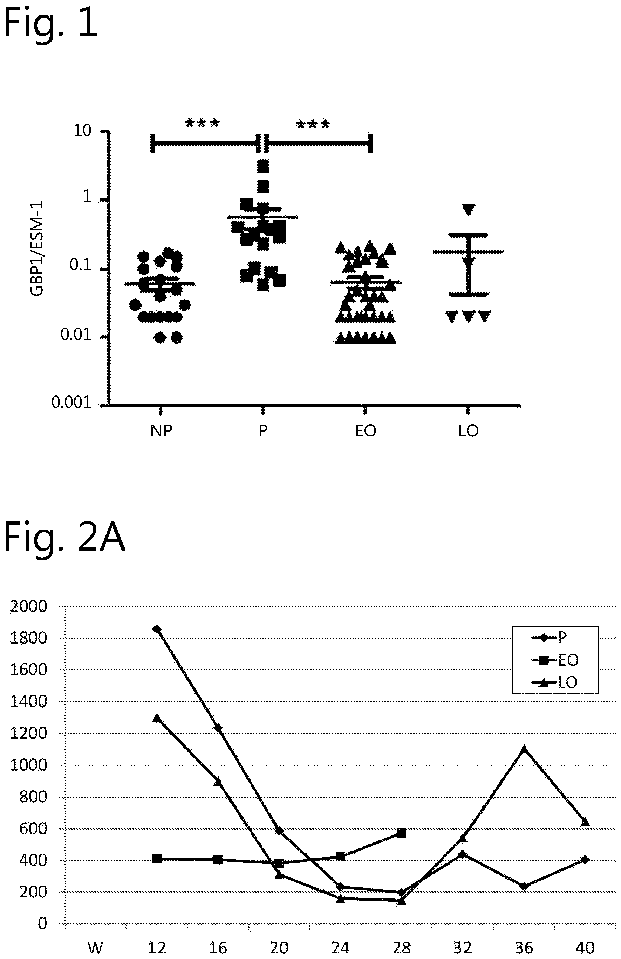

From the individual ELISA results obtained, the third trimester GBP-1/ESM-1 ratio was determined. This showed that for healthy pregnancies the ratio of GPB-1/ESM-1 was significantly increased compared to non-pregnant women or pregnancies complicated by early-onset preeclampsia. Although not significant the difference between healthy and late-onset preeclampsia most likely will be significant when a larger number of samples is available (Figure 1). Based on these results, we evaluated the GPB-1 / ESM-1 ratio over the complete gestational period starting from week 10 until term.

In a second experiment a total of 290 plasma samples from 23 healthy and GA matched control, 11 severe early-onset preeclampsia (PE) as well as 7 severe late onset PE pregnancies were collected over time at regular intervals between week 12±2 and birth. The GBP-1 and ESM-1 levels were assessed in all samples by ELISA.

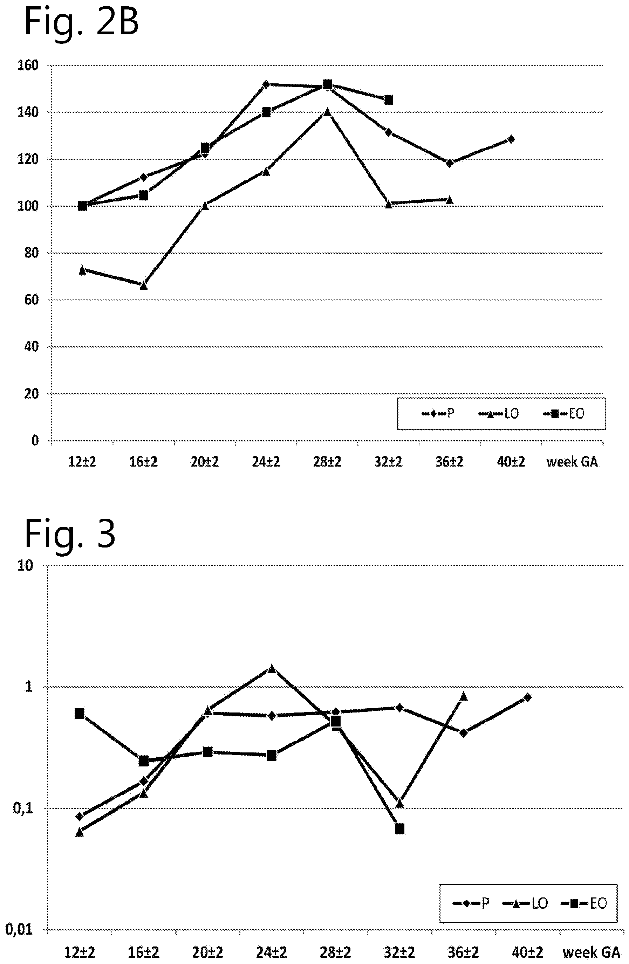

In healthy controls, the ESM-1 levels decrease between week 12±2 and birth (figure 2a), while GBP-1 levels increase during the same period (Figure 2b). In pregnancies that develop late-onset preeclampsia an initial reduction of ESM-1 was observed until week 20 similar to the decrease in healthy pregnancies. After week 20 a rapid increase of ESM-1 was observed concomitant with the manifestation of late-onset preeclampsia. In the same samples we observed an increase of the GBP-1 level after week 12±2 of gestation and remained stable until birth. In pregnancies that will develop early-onset preeclampsia, the ESM-1 levels are already significantly lower at week 12±2 and increase at the 'onset' of the syndrome. Surprisingly the GBP-1 level is increasing during the first period between about week 12 and 20.

The data of Fig. 2a are also provided in below table:

| **Week** | **pregnant** | **early-onset** | **late-onset** |
|---|---|---|---|
| 12±2 | 1857 | 410 | 1298 |
| 16±2 | 1235 | 405 | 899 |
| 20±2 | 586 | 382 | 312 |
| 24±2 | 233 | 423 | 159 |
| 28±2 | 198 | 573 | 148 |
| 32±2 | 438 | | 543 |
| 36±2 | 236 | | 1103 |
| 40±2 | 405 | | 646 |

The data of Fig. 2b are also provided in below table:

| **Week GA** | **Pregnant** | **Late-onset** | **Early-onset** |
|---|---|---|---|
| **12±2** | 100 | 73 | 100 |
| **16±2** | 112 | 67 | 105 |
| **20±2** | 122 | 101 | 125 |
| **24±2** | 152 | 115 | 140 |
| **28±2** | 151 | 141 | 152 |
| **32±2** | 132 | 101 | 145 |
| **36±2** | 118 | 103 | |
| **40±2** | 129 | | |

The concentrations in Figs. 2a and 2b and above tables related to these figures 2a and 2b are the means of the concentrations of respectively ESM-1 and GBP-1 in pg/ml measured in each of the groups.

Using these data, the individual GBP-1 / ESM-1 ratio was assessed for all the samples belonging to each time point and experimental group. This, in turn, was used to calculate the median ratio which was plotted against the gestational period from week 12 until birth (figure 3). The ratio of healthy pregnancies was 0.086 and those that developed severe late-onset PE 0.065. These are comparable, but in those pregnancies that develop severe early-onset PE, the ratio 0.604 was significantly higher as compared to healthy pregnancies. These results indicate that the ratio between GBP-1 and ESM-1 is a prognostic parameter to identify the women at risk for severe early-onset PE already as early as 12±2 weeks of gestation (Figure 3). In addition to a rule in parameter, the GBP-1 / ESM-1 ratio also serves as a rule out parameter. The data in Figure 3 are also provided in below table:

| **Week GA** | **Pregnant** | **Late-onset** | **Early-onset** |
|---|---|---|---|
| 12±2 | 0,086 | 0,065 | 0,604 |
| 16±2 | 0,167 | 0,135 | 0,245 |
| 20±2 | 0,607 | 0,649 | 0,29 |
| 24±2 | 0,579 | 1,433 | 0,273 |
| 28±2 | 0,619 | 0,481 | 0,522 |
| 32±2 | 0,673 | 0,112 | 0,068 |
| 36±2 | 0,418 | 0,845 | |
| 40±2 | 0,819 | | |

In the below table, the early-onset (EO) and late-onset (LO) ratios are divided by the pregnant ratios (P):

| **week GA** | **EO/P** | **LO/P** |
|---|---|---|
| 12±2 | 7,023 | 0,756 |
| 16±2 | 1,467 | 0,808 |
| 20±2 | 0,478 | 1,069 |
| 24±2 | 0,472 | 2,475 |
| 28±2 | 0,845 | 0,778 |
| 32±2 | 0,101 | 0,166 |
| 36±2 | | 2,022 |
| 40±2 | | |

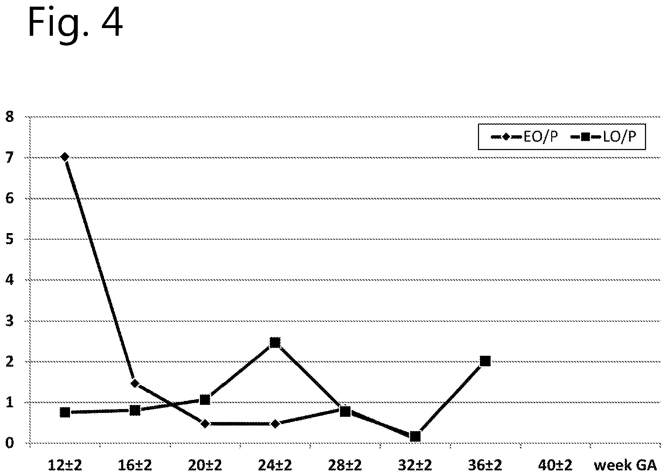

The EO/P and LO/P ratios are also displayed in Fig. 4.

Especially, the ratios can be used in those weeks wherein the ratios differ by a factor of at least 2, even more especially at least by a factor 3, yet even more especially by a factor 4. Hence, for early onset preeclampsia, especially weeks 8-18, more especially 10-18, such as week 12-16 can be used for the identification. This is especially herein also indicated as weeks 12±2 - 16±2 of gestation.

It is envisaged that in weeks 8-10 the same trend may be visible.

Hence, in an aspect the invention provides an in vitro method for identifying from a biological fluid sample from a pregnant subject a pregnancy related syndrome selected from the group consisting of Preeclampsia, Eclampsia, and Hemolysis Elevated Liver enzymes and Low Platelets (HELLP), as further defined in the accompanying claims. Solely disclosed is a method of treatment of a subject being at risk of developing a pregnancy related syndrome, comprising typing the subject according to the methods as described herein, and treating the subject with low dose Aspirin. A method of treatment of a subject being at risk of developing a pregnancy related syndrome is also disclosed and comprises typing the subject according to the methods as described herein, and treating the subject in a way the ratio between GBP-1 and ESM-1 decreases by lowering the GBP-1 levels. For instance, such methods of treatment may be for a period of at least four weeks.

As indicated above, both GBP-1 and ESM-1 play a role in the regulation of angiogenesis and are released by activated endothelial cells. For ESM-1 we have shown the concentrations at different time points during gestation. Here it was tested whether GBP-1 is increased in preeclampsia (PE) and whether the ratio between GBP-1 and ESM-1 is informative during gestation. Plasma samples from high risk pregnancies divided in 23 healthy, 11 severe early-onset PE and 7 severe late-onset PE pregnancies were collected at regular intervals between week 12±2 and birth. GBP-1 and ESM-1 were both measured by ELISA. Surprisingly, the ratio between GBP-1 and ESM-1 differed between the three groups at week 12±2. Although this ratio was comparable for women with healthy pregnancies and those that developed severe late-onset PE, the GBP-1/ESM-1 ratio was significantly increased between women that develop severe early-onset PE and healthy control pregnancies. This indicates that the ratio between GBP-1 and ESM-1 is a prognostic parameter to identify the women at risk for severe early-onset PE already as early as 12±2 weeks of gestation. This ratio serves, therefore, both as an important rule-in and rule out parameter. The increased GBP-1/ESM-1 ratio that as observed in women that will develop preeclampsia is probably caused by endothelial cell activation in these conditions.

### BRIEF DESCRIPTION OF THE DRAWINGS

Figure 1 shows third trimester GBP-1/ESM-1 ratios (y-axis (logarithmic scale)) in plasma of healthy pregnant (n=32), indicated with P, severe early-onset preeclampsia (n=52), indicated with EO, and severe late-onset (n=8) preeclampsia, indicated with LO, and from non pregnant women (n=25), indicated with NP. Significance calculated by Mann Whitney test: *** = p<0.0001;
Figure 2a and b show ESM-1 (Figure 2a) and GBP-1 (Figure 2b) concentrations in plasma during the pregnancy (week 12±2 until birth) of healthy pregnant (n=23), indicated with P, severe early-onset preeclampsia (n=11), indicated with EO, and severe late-onset preeclampsia (n=7), and indicated with LO; and
Figure 3 shows the ratio between GBP-1 and ESM-1 in plasma during pregnancy (week 12±2 until birth) of healthy pregnant (n=23), indicated with P, severe early-onset preeclampsia (n=11), indicated with EO, and severe late-onset preeclampsia (n=7), indicated with PO. On the x-axis the weeks (W) are displayed, and on the y-axis the ratio GBP-1 and ESM-1.

### DETAILED DESCRIPTION OF THE EMBODIMENTS

In an aspect the invention provides an in vitro method for identifying from a biological fluid sample from a pregnant subject a pregnancy related syndrome selected from the group consisting of Preeclampsia, Eclampsia, and Hemolysis Elevated Liver enzymes and Low Platelets (HELLP), wherein the biological fluid sample comprises a blood sample, a plasma sample, or a serum sample from the pregnant subject, the method including (i) determining a first parameter that scales with the concentration of GBP-1 in said biological fluid sample and a second parameter that scales with the concentration of ESM-1 in said biological fluid sample, and defining a ratio of the first parameter and the second parameter; (ii) comparing this ratio of the first and the second parameter with a reference value, wherein the reference value is a reference ratio of GBP-1 and ESM-1 of a subject having a healthy pregnancy; and (iii) identifying the subject as being likely to have or develop the pregnancy related syndrome based on the comparison of the ratio of the first parameter and the second parameter and the reference ratio.

In yet a further aspect, the invention provides such a method including
a. measuring the concentrations of GBP-1 and ESM-1 in the biological sample, wherein the biological sample is from the pregnant subject being in any one of week 10-36;
b. determining the ratio between the concentration of GBP-1 and the concentration of ESM-1;
c. comparing said ratios to the reference value, and
d. identifying the subject as being likely to develop the pregnancy related syndrome based on a comparison of the ratio between the concentrations GBP-1 and ESM-1 to the reference value.

Such method may especially be applied to determine whether or not it may be likely that the subject is being likely to develop severe early-onset preeclampsia.

In yet a further aspect, the invention provides a method for identifying from a biological fluid sample from a pregnant subject a pregnancy related syndrome selected from the group consisting of Preeclampsia, Eclampsia, and HELLP comprising the stages of
a. measuring the concentrations of GBP-1 and ESM-1 in the biological sample, wherein the biological sample is from the pregnant subject being in any one of week 10-16 of gestation;
b. determining the ratio such as especially by dividing the concentration of GBP-1 by the concentration of ESM-1;
c. comparing said ratios to the reference value, and
d. identifying the subject as being likely to have the pregnancy related syndrome based on a comparison of the ratio between the concentrations GBP-1 and ESM-1 to the reference value when said ratio between the concentrations GBP-1 and ESM-1 is larger than the reference value.

Such method may especially be applied to determine whether or not it may be likely that the subject is being likely to develop severe early-onset preeclampsia.

In yet a further aspect, the invention provides the method for identifying from a biological fluid sample from a pregnant subject a pregnancy related syndrome selected from the group consisting of Preeclampsia, Eclampsia, and HELLP comprising the stages of
a. measuring the concentrations of GBP-1 and ESM-1 in the biological sample, wherein the biological sample is from the pregnant subject being in any one of week 10-18 of gestation and wherein said biological fluid sample comprises a plasma or serum sample;
b. determining the ratio such as especially by dividing the concentration of GBP-1 by the concentration of ESM-1;
c. comparing said ratios to a reference value, and
d. identifying the subject to have or develop the pregnancy related syndrome when the ratio between the concentrations GBP-1 and ESM-1 is in the range of 7±3; as further defined in the accompanying claims.

Such method may especially be applied to determine whether or not it may be likely that the subject is being likely to develop severe early-onset preeclampsia.

In yet a further aspect, the invention provides the method for identifying from a biological fluid sample from a pregnant subject a pregnancy related syndrome selected from the group consisting of Preeclampsia, Eclampsia, and HELLP wherein the subject is identified as having or developing the pregnancy-related syndrome when the ratio is equal to or larger than 400% (i.e. at least 4 times) of the ratio between the concentrations GBP-1 and ESM-1 of biological fluid samples of pregnant subjects having a healthy pregnancy; as further defined in the accompanying claims.

Such method may especially be applied to determine whether or not it may be likely that the subject is being likely to develop severe early-onset preeclampsia.

In an embodiment, said stage(s) of measuring are done using one or more immunological assays, especially wherein the one or more immunological assay comprise in ELISA (Enzyme-linked immunosorbent assay) assay, a turbidimetric assay, radioimmunosorbent assay (RIST), a radioimmunoassay (RIA), a direct or indirect immunofluorescence assay, a particle gel immuno assay (PaGIA), or a lateral flow assay. In an embodiment, said immunological assay(s) are an ELISA. In an embodiment, said immunological assay(s) are a lateral flow assay. In an embodiment, said stage(s) of measuring are done using one or more enzymatic calorimetric assay(s). In an embodiment, said stage(s) of measuring are done using mass spectrometry (MS).

In one embodiment, the method further comprises the stage of uterine artery Doppler screening and deriving thereof information about the placentation.

In an embodiment, said concentration of GBP-1 and ESM-1 is the level of free GBP-1, free ESM-1 or tthe level of GBP-1 and/or ESM-1 bound to or complexed with their respective agonist in the biological sample.

In a further aspect, the invention provides a device for identifying from a biological fluid sample from a pregnant subject a pregnancy related syndrome selected from the group consisting of Preeclampsia, Eclampsia, and HELLP said device comprising (a) an analyzing unit configured to determine a first parameter that scales with the concentration of GBP-1 in said biological fluid sample and a second parameter that scales with the concentration of ESM-1 in said biological fluid sample; (b) an evaluation unit comprising a data processor having implemented necessary algorithms for determining a ratio between the first parameter and the second parameter and comparing the ratio to a reference value stored in a database in order to determine whether a pregnant subject is likely to have or develop preeclampsia, eclampsia, and/or HELLP wherein especially the reference value is a reference ratio of GBP-1 and ESM-1 of a subject having a healthy pregnancy; as further defined in the accompanying claims. The term "analysing unit" may also refer to a plurality of (different) analysing units.

In yet a further aspect, the invention provides a device for identifying a pregnant subject that is likely to have or develop preeclampsia, eclampsia, and/or HELLP said device comprising:
a. An analyzing unit comprising a detection agent for GBP-1 and one for ESM-1, for instance being an antibody, a (recombinant) receptor or an aptamer, which allows the determination of the concentrations of GBP-1 and ESM-1;
b. An evaluation unit comprising a data processor having implemented necessary algorithms for calculation of the ratio between the concentration of GBP-1 and ESM-1 and comparing the ratio determined with reference values stored in a database in order to determine whether a pregnant subject is likely to have or develop preeclampsia, eclampsia, and/or HELLP; as further defined in the accompanying claims.

Disclosed is a device comprising:
a. An analyzing unit comprising a detection agent for GBP-1, ESM-1, sFlt1, VEGF, P1GF, HGF, sEndoglin, pp-13, PAPP-A, GDF-15, pikachuring or hemopexin, which allows the determination of the concentration of GBP-1, ESM-1, sFlt1, VEGF, P1GF, HGF, sEndoglin, pp-13, PAPP-A, GDF-15, pikachuring or hemopexin.
b. An evaluation unit comprising a data processor having implemented necessary algorithms for determining ratios and comparing the concentrations and/or ratios of GBP-1, ESM-1, sFlt1, VEGF, P1GF, HGF, sEndoglin, pp-13, PAPP-A, GDF-15, pikachuring and hemopexin determined with reference values stored in a database and calculate the ratio between the measured markers in order to determine whether a pregnant subject is likely to have or develop preeclampsia, eclampsia and/or HELLP; as further defined in the accompanying claims.

In an embodiment, the device may further comprise an analyzing unit comprising a detection agent for sFlt1, VEGF, P1GF, HGF, sEndoglin, pp-13, PAPP-A, GDF-15, pikachuring or hemopexin, which allows the determination of the concentration of sFlt1, VEGF, P1GF, HGF, sEndoglin, pp-13, PAPP-A, GDF-15, pikachuring or hemopexin, and an evaluation unit comprising a data processor having implemented necessary algorithms for comparing the concentration of sFlt1, VEGF, P1GF, HGF, sEndoglin, pp-13, PAPP-A, GDF-15, pikchurin or hemopexin determined with reference values stored in a database and calculate the ratio between the measured markers in order to determine whether a pregnant subject is likely to have or develop preeclampsia, eclampsia, HELLP and/or IUGR, and wherein said evaluation unit is also capable of giving therapeutic recommendations.

In an embodiment, said evaluation unit is also capable of giving therapeutic recommendations.

In yet a further aspect, the invention provides a use of an evaluation of the concentrations of and ratio between GBP-1 and ESM-1 in an extracted biological sample from a pregnant subject for identifying whether the pregnant subject is likely to have or develop preeclampsia, eclampsia and/or HELLP; as further defined in the accompanying claims.

In one embodiment, the pregnant subject is in any one of week 10-36, such as week 10-18 of gestation.

In an aspect, the invention provides a kit comprising an ELISA for GBP-1 and an ELISA for ESM-1; as further defined in the accompanying claims. Yet further, the invention provides a kit for identifying whether a pregnant subject being in any one of week 10-36 gestation is likely to have or develop a pregnancy related syndrome selected from the group consisting of Preeclampsia, Eclampsia, and HELLP said kit comprising: (a) an analyzing unit comprising a detection agent for ESM-1 and an analyzing unit comprising a detection agent for GBP-1; and a detection agent for one or more of sF1t1, VEGF, P1GF, HGF, sEndoglin, pp-13, PAPP-A, GDF-15, pikachurin and hemopexin; (b) an evaluation unit for determining whether a pregnant subject is likely to have or develop the pregnancy related syndrome, based on a result provided by the analyzing unit, especially comprising an evaluation unit comprising a data processor having implemented necessary algorithms for comparing the concentrations of GBP-1 and ESM-1, or their ratio, to one or more of sFlt1, VEGF, P1GF, HGF, sEndoglin, pp-13, PAPP-A, GDF-15, pikachurin and hemopexin determined with reference values stored in a database and calculate the ratio between the measured markers in order to determine whether a pregnant subject is likely to have or develop the pregnancy related syndrome; as further defined in the accompanying claims.

In an embodiment, the kit may further comprise a manual or a reference to a (remote) manual. In an embodiment, the manual includes instructions how to extract biological sample from a pregnant subject and/or how to use the analyzing unit and/or how to use the evaluation unit. In an embodiment, the evaluation unit comprises a color scheme, wherein the analyzing unit is configured to provide a color reaction wherein the color is dependent upon the concentration of GBP-1 and/or ESM-1 in an extracted biological sample from a pregnant subject.

In an embodiment, said evaluation unit is also capable of giving therapeutic recommendations.

In an embodiment, the analyzing unit comprises an assay. In an embodiment, the analyzing unit comprises an ELISA (Enzyme-linked immunosorbent assay) assay, a turbidimetric assay, radioimmunosorbent assay (RIST), radioimmunoassay (RIA), direct or indirect immunofluorescence, particle gel immuno assay (PaGIA), mass spectrometry (MS) or lateral flow assay.

In an embodiment, the manual includes information to extract biological sample from a pregnant subject in any one week of gestation, especially between week 1 to 20 of gestation, even more especially between weeks 10 to 16. Especially, the biological sample is from a pregnant subject in any one week 8-20 of gestation, such as 10-20 of gestation, even more especially week 8-18 of gestation, like 10-18, such as week 10-16, especially like week 10-14. For instance, the biological sample is from a pregnant subject in any one week 12-16, such as week 12-14, of gestation.

In an aspect, the invention provides the use of such a kit for predicting a pregnant subject in any one of week 10-36, such as week 10-18 of gestation to develop after week 20 severe early-onset preeclampsia, based on a measurement of the concentrations of GBP-1 and/or ESM-1 or the ratio between the two in a biological fluid sample from the pregnant subject, wherein the biological fluid sample comprises a blood sample, a plasma sample, or a serum sample from said pregnant subject.

In an embodiment, the manual includes information to extract biological sample from a pregnant subject in any one week of gestation, especially until week 32 of gestation, such as in week 22-30, like week 22-28 of gestation

The second occasion is especially later in time than the first occasion. Further, the method and use, as well as the application of the kit may include extracting one biological sample, two biological samples, but also more than two biological samples. Especially, these are taken at different times, such as with one or more days in between, or one or more weeks in between.

The method and use, as well as the application of the kit may include taking a plurality of samples over a period of time and determining with the GBP-1 and ESM-1 specific assays the concentration(s) of GBP-1 and/or ESM-1 or derivative values thereof like ratios of values. Alternative or additionally, the quantification is an indirect quantification, for instance by a color reaction that may be dependent upon the concentration (concentration). Based on the color, a prediction may be made about a pregnant subject whether she is likely to have or develop preeclampsia, eclampsia, and/or Hemolysis Elevated Liver enzymes and Low Platelets (HELLP).

Additionally or alternatively, reference values may be determined or provided, for instance in a manual (on the internet) based upon one can determine whether the pregnant subject has or is likely to develop one of the above-mentioned syndromes. When comparing the value obtained of the subject with a reference value, or a plurality of reference values when more than one parameter is determined, the status of likely status to be can be predicted.

Especially, the invention provides a kit, a method, or a device; as further defined in the accompanying claims. Such method may include a number of actions. Further, the kit, or device may be used (in a method) to execute one or more of these actions. The kit, a method, or a device may especially be used to determine from a biological fluid sample from a pregnant subject a pregnancy related syndrome, such as mentioned herein, especially preeclampsia, even more especially severe early onset preeclampsia.

The actions may especially include (in a determination stage) determining a first parameter that scales with the concentration of GBP-1 in said biological fluid sample and a second parameter that scales with the concentration of ESM-1 in said biological fluid sample. The determination stage may further include defining a ratio of the first parameter (GBP-1) and the second parameter (ESM-1).

The actions may further comprise (in a comparison stage) comparing this ratio of the first and the second parameter with a reference ratio. The reference ratio can also be indicated as a reference value.

The term "reference ratio" may also refer to a plurality of reference ratios, such e.g. for a plurality of weeks of gestation, or a plurality of days of gestation. The term "reference value" may also refer to a plurality of reference values, such e.g. for a plurality of weeks of gestation, or a plurality of days of gestation.

The reference ratio is especially based on biological fluid samples of pregnant subjects that had healthy pregnancies. Hence, the reference ratio may especially be based on determining a first reference parameter that scales with the concentration of GBP-1 in biological fluid samples and a second reference parameter that scales with the concentration of ESM-1 in said biological fluid samples (of pregnant subjects that had healthy pregnancies).

Especially, the method to determine the first parameter and the first reference parameter are substantially identical, although in specific embodiments this may not necessarily be the case. Such embodiments may e.g. include embodiments wherein an internal reference is used, embodiments wherein absolute concentrations are measured and/or embodiments wherein trends in the ratios are compared.

Especially, the method to determine the second parameter and the second reference parameter are substantially identical, although in specific embodiments this may not necessarily be the case. Such embodiments may e.g. include embodiments wherein an internal reference is used, embodiments wherein absolute concentrations are measured and/or embodiments wherein trends in the ratios are compared.

Herein, especially the ratio is defined as the quotient of GBP-1 and ESM-1. The values given herein especially apply for these ratios. However, as will be clear to a person skilled in the art, also the quotient of ESM-1 and GBP-1 may be used; then the reciprocal values of the ratios given herein may be used. Hence, the phrase "determining the ratio between the concentrations GBP-1 and ESM-1 by dividing the GBP-1 concentration by the ESM-1 concentration" and similar phrases may alternatively in embodiments also refer to dividing the ESM-1 concentration by the GBP -1 concentration

The phrase "parameter that scales with the concentration" may refer to the concentration of the respective species. However, this phrase may also refer to e.g. a sensor signal, such as of a GC or a spectrometer, etc.. The parameter may especially linearly scale over at least part of the concentration range. The concentration range may be about 10-10000 pg/ml (for humans).

GBP-1 refers especially to interferon-induced guanylate-binding protein 1, which is a protein that in humans is encoded by the GBP1 gene. It belongs to the dynamin superfamily of large GTPases. ESM-1 refers especially to endothelial cell-specific molecule 1, which is a protein that in humans is encoded by the ESM1 gene. This gene encodes a secreted protein which is mainly expressed in the endothelial cells in human lung and kidney tissues.

GBP-1 and ESM-1 may also be indicated as "biomarker" or simply as "protein" or as "polypeptide". Herein, these may also be indicated as "prognostic parameters".

As described in WO2014/001244, the term "preeclampsia" especially refers to a medical condition which is characterized by hypertension and proteinuria. Preeclampsia occurs in pregnant female subjects and the hypertension is also referred to as pregnancy-induced hypertension. Preferably, the pregnancy-induced hypertension is identified to be present in a subject by two blood pressure measurements of 140 mmHg (systolic) and/or 90 mmHg (diastolic) or more, wherein said two measurements have been made at least 6 hours apart. Proteinuria is, preferably, identified to be present by 300 mg protein or more in a 24-hour urine sample. Preeclampsia may progress to eclampsia, a life-threatening disorder characterized by the appearance of tonic-clonic seizures or coma conditions. Symptoms associated with severe preeclampsia are oligouria of less than 500 ml within 24 hours, cerebral or visual disturbance, pulmonary edema or cyanosis, epigastric- or right upper quadrant- pain, impaired liver function, thrombocytopenia, fetal growth restriction. Subjects suffering from preeclampsia with hepatic involvement may further develop the HELLP syndrome. Accordingly, a subject according to the invention which is at risk of developing preeclampsia, preferably, is also potentially at risk of developing the HELLP syndrome. The HELLP syndrome is associated with a high risk of adverse outcomes such as placental abruption, renal failure, subcapsular hepatic hematoma, recurrent preeclampsia, preterm delivery, or even mater al and/or fetal death. Further details of preeclampsia and the accompanying symptoms as well as the follow up diseases such as HELLP syndrome or eclampsia are to be found in standard text books of medicine or Guidelines of the relevant medical societies. Details can be found, e.g., in ACOG Practice Bulletin, Clinical Management Guidelines for Obstetrician - Gynecologists, no.: 33, January 2002 or Leitlinien, Empfehlungen, Stellungnahmen of the Deutschen Gesellschaft fur Gynakologie und Geburtshilfe e.V., August 2008, NICE Clinical Guideline Hypertension in pregnancy: the management of hypertensive disorders during pregnancy, August 2010 (revised reprint January 2011).

The terms "GBP-1" and "ESM-1" may each independently also encompasses variants of these (human) polypeptides, respectively. Such variants have at least the same essential biological and immunological properties as the respective polypeptide. In particular, they share the same essential biological and immunological properties if they are detectable by the same specific assays referred to in this specification, e.g., by ELISA assays using polyclonal or monoclonal antibodies specifically recognizing the respective polypeptides. Moreover, such variant may have an amino acid sequence which differs due to at least one amino acid substitution, deletion and/or addition wherein the amino acid sequence of the variant is still, preferably, at least 50%, even more especially at least 70%, yet even more especially at least 85%, like at least 90%, such as especially at least 95%, like even more especially at least 98% identical with the amino sequence of the respective polypeptide. The degree of being identical between two amino acid sequences can be determined by algorithms well known in the art.

The actions may further comprise (in an identification stage) identifying the subject as being likely to have or develop the pregnancy related syndrome based on the comparison of the ratio of the first parameter and the second parameter and the reference ratio.

The actions may thus further also comprise (in an identification stage) identifying the subject as being likely to have or develop a healthy pregnancy (in view of the pregnancy related syndrome) based on the comparison of the ratio of the first parameter and the second parameter and the reference ratio.

As indicated above, when the ratio is larger than the reference ratio in a biological fluid sample from a pregnant subject in week 1-20, especially week 8-18, like week 10-16, especially like week 12-14 of gestation, then the subject may be identified as being likely to develop (or likely to have) the pregnancy related syndrome, especially preeclampsia, even more especially severe early-onset preeclampsia.

As indicated above, when the ratio is smaller than the reference ratio in a biological fluid sample from a pregnant subject in week 20-36, especially week 22-30 of gestation, then the subject may be identified as being likely to develop (or likely to have) the pregnancy related syndrome, especially preeclampsia, even more especially severe early-onset preeclampsia. Alternatively, the subject may be identified as being likely to develop (or likely to have) severe late-onset preeclampsia.

A human pregnancy counts in average 40 weeks of gestation.

Hence, for ESM-1 we have shown the concentrations at different time points during gestation. Here it was tested whether GBP-1 is increased in preeclampsia (PE) and whether the ratio between GBP-1 and ESM-1 is informative during gestation. Plasma samples from high risk pregnancies divided in 23 healthy, 11 severe early-onset PE and 7 severe late-onset PE pregnancies were collected at regular intervals between week 12±2 and birth. GBP-1 and ESM-1 were both measured by ELISA. Surprisingly, the ratio between GBP-1 and ESM-1 differed between the three groups at week 12±2. Although this ratio was comparable for women with healthy pregnancies and those that developed severe late-onset PE, the GBP-1/ESM-1 ratio was significantly increased between women that develop severe early-onset PE and healthy control pregnancies. This indicates that the ratio between GBP-1 and ESM-1 is a prognostic parameter to identify the women at risk for severe early-onset PE already as early as 12±2 weeks of gestation. This ratio serves, therefore, both as an important rule-in and rule-out parameter. The increased GBP-1/ESM-1 ratio that as observed in women that will develop early- onset preeclampsia is probably caused by endothelial cell activation in these conditions.

The term "substantially" herein, such in "substantially consists", will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of'. The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. However, the terms first and second, etc., may also indicate a relation in time. For instance, a first sample may be extracted earlier in time than a second sample. Especially, this applies to the terms "first occasion" and "second occasion". Based on the measured values, a diagnosis may be made.

The devices herein may amongst others described during operation. As will be clear to the person skilled in the art, the invention is not limited to methods of operation or devices in operation.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "to comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

The invention further applies to a device comprising one or more of the characterizing features described in the description and/or shown in the attached drawings. The invention further pertains to a method or process comprising one or more of the characterizing features described in the description and/or shown in the attached drawings.

## Claims

1. An in vitro method for identifying from a biological fluid sample from a pregnant subject a pregnancy related syndrome selected from the group consisting of Preeclampsia, Eclampsia, and Hemolysis Elevated Liver enzymes and Low Platelets (HELLP), wherein the biological fluid sample comprises a blood sample, a plasma sample, or a serum sample from the pregnant subject, the method including
(i) determining a first parameter that scales with the concentration of GBP-1 in said biological fluid sample and a second parameter that scales with the concentration of ESM-1 in said biological fluid sample, and defining a ratio of the first parameter and the second parameter;
(ii) comparing this ratio of the first and the second parameter with a reference value, wherein the reference value is a reference ratio of GBP-1 and ESM-1 of a subject having a healthy pregnancy; and
(iii) identifying the subject as being likely to have or develop the pregnancy related syndrome based on the comparison of the ratio of the first parameter and the second parameter and the reference ratio.

2. The in vitro method according to claim 1, the method including
(ia) measuring the concentrations of GBP-1 and ESM-1 in the biological sample, wherein the biological sample is from the pregnant subject being in any one of week 10-36;
(ib) determining the ratio between the concentrations of GBP-1 and ESM-1;
(ii) comparing said ratio to the reference value, and
(iii) identifying the subject as being likely to have or develop the pregnancy related syndrome based on a comparison of the ratio between the GBP-1 and ESM-1 concentrations to the reference value.

3. The method according to any one of the preceding claims, wherein determining the ratio between the concentrations GBP-1 and ESM-1 comprises dividing the GBP-1 concentration by the ESM-1 concentration, wherein
- the method further comprising (iii) identifying the subject, especially being in any one of week 10-16 of gestation, as being likely to develop the pregnancy related syndrome based on a comparison of the ratio between the concentrations GBP-1 and ESM-1 to the reference value when said ratio between the concentrations GBP-1 and ESM-1 is larger than the reference value; and/or
- said biological fluid sample is from a pregnant subject being in any one week of gestation between weeks 10-18, and wherein said biological fluid sample comprises a plasma or serum sample and wherein the pregnant subject is evaluated to have the pregnancy related syndrome when the ratio between the concentrations GBP-1 and ESM-1 is in the range of 7±3.

4. The method according to any one of the preceding claims, wherein the ratio between the concentrations GBP-1 and ESM-1 of the biological fluid sample of the pregnant subject is indicative of having or developing the pregnancy related syndrome when the ratio is equal to or larger than 400% of the ratio between the concentrations GBP-1 and ESM-1 of biological fluid samples of pregnant subjects having a healthy pregnancy.

5. The method according to any one of the preceding claims, further comprising using an assay specific to GBP-1 or ESM-1, wherein the assay comprises an ELISA (Enzyme-linked immunosorbent assay) assay, a turbidimetric assay, radioimmune sorbent assay (RIST), a radioimmunoassay (RIA), a direct or indirect immunofluorescence assay, a particle gel immuno assay (PaGIA), or a lateral flow assay, especially wherein the assay comprises one or more of an ELISA, a lateral flow assay, an enzymatic assay, a colorimetric assay, and a luminescent assay, and/or wherein measuring the concentration of ESM-1 in the biological sample includes using mass spectrometry (MS); the method further comprising the stage of uterine artery Doppler screening and deriving thereof information about the placentation.

6. The method according to any one of the preceding claims, wherein said concentration of GBP-1 and ESM-1 is the level of free GBP-1 and/or free ESM-1 in the biological sample, and/or wherein said concentration of GBP-1 and ESM-1 is the level of GBP-1 and/or ESM-1 bound to or complexed with their respective agonist in the biological sample.

7. The method according to any one of the preceding claims, wherein one or more of (a) measuring the concentration of GBP-1 and/or ESM-1 in the biological sample comprises a method to do a quantification of RNA coding for GBP-1 and/or ESM-1 in cells or cell-free RNA in the biological sample, and wherein (b) measuring the concentration of GBP-1 and/or ESM-1 in the biological sample comprises a method to quantify the concentration of protein via the concentration of RNA coding for ESM-1; and wherein said quantification is done via RT-PCR, PCR, hybridization or other means to determine the concentration of nucleotides.

8. The method according to any one of the preceding claims, the method comprising providing a first biological sample from a subject extracted on a first occasion, and providing a second biological sample from the subject extracted on a second occasion, measuring the concentration of GBP-1 and ESM-1 in the first biological sample, constituting a first concentration, using an assay specific to GBP-1 and ESM-1, measuring the concentration of GBP-1 and ESM-1 in the second biological sample, and constituting a second concentration, and the method further comprising identifying the subject as being likely to have or develop the pregnancy related syndrome based on a comparison of the first ratio between the two markers from the first concentrations and the ratio between the two markers from the second concentrations.

9. A device for identifying from a biological fluid sample from a pregnant subject a pregnancy related syndrome selected from the group consisting of Preeclampsia, Eclampsia, and Hemolysis Elevated Liver enzymes and Low Platelets (HELLP), wherein the biological fluid sample comprises a blood sample, a plasma sample, or a serum sample from the pregnant subject, said device comprising (a) an analyzing unit configured to determine a first parameter that scales with the concentration of GBP-1 in said biological fluid sample and a second parameter that scales with the concentration of ESM-1 in said biological fluid sample; (b) an evaluation unit comprising a data processor having implemented necessary algorithms for determining a ratio between the first parameter and the second parameter and comparing the ratio to a reference value stored in a database in order to determine whether a pregnant subject is likely to have or develop preeclampsia, eclampsia, and/or HELLP, wherein the reference value is a reference ratio of GBP-1 and ESM-1 of a subject having a healthy pregnancy.

10. The device according to claim 9, for identifying a pregnant subject being in any one of week 10-36 of gestation that is likely to have or develop a pregnancy related syndrome selected from the group consisting of Preeclampsia, Eclampsia, and HELLP, said device comprising (a) an analyzing unit comprising detection agents for GBP-1 and ESM-1, for instance being an combination of antibodies specific for GBP-1 and/or ESM-1, a (recombinant) receptor specific for GBP-1 and/or ESM-1 or an aptamer specific for GBP-1 and/or ESM-1, which allows the determination of the concentrations of GBP-1 and ESM-1; (b) an evaluation unit comprising a data processor having implemented necessary algorithms for determining the individual concentrations, the ratio between the concentrations and comparing the ratio to a reference value stored in a database in order to determine whether a pregnant subject is likely to have or develop preeclampsia, eclampsia, and/or HELLP.

11. The computer-implemented use of an evaluation of the concentration of GBP-1 and ESM-1 or their ratio in an extracted biological fluid sample from a pregnant subject being in any one of week 10-36, such as week 10-18 of gestation, for identifying whether the pregnant subject is likely to have or develop a pregnancy related syndrome selected from the group consisting of Preeclampsia, Eclampsia, and HELLP, wherein the biological fluid sample comprises a blood sample, a plasma sample, or a serum sample from the pregnant subject.

12. In vitro use of an extracted biological fluid sample from a pregnant subject being in any one of week 10-36 of gestation for identifying whether the pregnant subject is likely to have or develop a pregnancy related syndrome selected from the group consisting of Preeclampsia, Eclampsia, and HELLP, wherein the biological fluid sample comprises a blood sample, a plasma sample, or a serum sample from the pregnant subject, the use comprising: (a) measuring the concentrations of GBP-1 and ESM-1 in the biological sample; (b) comparing said concentrations of GBP-1 and ESM-1 or their ratio to a reference value; and (c) identifying the subject as being likely to have or develop the pregnancy related syndrome.

13. A kit for determining from a biological fluid sample from a pregnant subject a pregnancy related syndrome selected from the group consisting of Preeclampsia, Eclampsia, and HELLP, wherein the biological fluid sample comprises a blood sample, a plasma sample, or a serum sample from the pregnant subject the kit comprising an ELISA for GBP-1, an ELISA for ESM-1, and a manual or a reference to a manual providing reference values based upon one can determine whether the pregnant subject has or is likely to develop one of the pregnancy related syndromes.

14. A kit for identifying whether a pregnant subject being in any one of week 10-36 gestation is likely to have or develop a pregnancy related syndrome selected from the group consisting of Preeclampsia, Eclampsia, and HELLP, said kit comprising: (a) an analyzing unit comprising a detection agent for ESM-1 and an analyzing unit comprising a detection agent for GBP-1; and a detection agent for one or more of sFlt1, VEGF, P1GF, HGF, sEndoglin, pp-13, PAPP-A, GDF-15, pikachurin and hemopexin; (b) an evaluation unit for determining whether a pregnant subject is likely to have or develop the pregnancy related syndrome, based on a result provided by the analyzing unit, especially comprising an evaluation unit comprising a data processor having implemented necessary algorithms for comparing the concentrations of GBP-1 and ESM-1, or their ratio, to one or more of sFlt1, VEGF, P1GF, HGF, sEndoglin, pp-13, PAPP-A, GDF-15, pikachurin and hemopexin determined with reference values stored in a database and calculate the ratio between the measured markers in order to determine whether a pregnant subject is likely to have or develop the pregnancy related syndrome.

15. The kit according to claim 14, further comprising a manual or a reference to a manual, wherein the manual includes instructions how to extract biological fluid sample from a pregnant subject and/or how to use the analyzing unit and/or how to use the evaluation unit, wherein the manual includes information to extract biological fluid sample from a pregnant subject in any one of week 10-36 of gestation, and wherein the evaluation unit comprises a color scheme, wherein the analyzing unit is configured to provide a color reaction wherein the color is dependent upon the ratio based on the concentrations of GBP-1 and ESM-1 in an extracted biological sample from a pregnant subject.

16. Use of a kit according to any one of claims 13-15, for predicting a pregnant subject in any one of week 10-36, such as week 10-18 of gestation to develop after week 20 severe early-onset preeclampsia, based on a measurement of the concentrations of GBP-1 and ESM-1 or the ratio between the two in a biological fluid sample from the pregnant subject, wherein the biological fluid sample comprises a blood sample, a plasma sample, or a serum sample from said pregnant subject.

## Patentansprüche

1. In-vitro-Verfahren zum Identifizieren eines schwangerschaftsbedingten Syndroms, das aus der Gruppe bestehend aus Präeklampsie, Eklampsie, und Hämolyse erhöhten Leberenzymen und niedriger Thrombozytenzahl (HELLP) ausgewählt wird, aus einer biologischen Flüssigkeitsprobe von einem schwangeren Individuum, wobei die biologische Flüssigkeitsprobe eine Blutprobe, eine Plasmaprobe oder eine Serumprobe vom schwangeren Individuum umfasst, wobei das Verfahren umfasst:
(i) Bestimmen eines ersten Parameters, der mit der Konzentration von GBP-1 in der biologischen Flüssigkeitsprobe skaliert, und eines zweiten Parameters, der mit der Konzentration von ESM-1 in der biologischen Flüssigkeitsprobe skaliert, und Definieren eines Verhältnisses des ersten Parameters und des zweiten Parameters;
(ii) Vergleichen dieses Verhältnisses des ersten und des zweiten Parameters mit einem Referenzwert, wobei der Referenzwert ein Referenzverhältnis von GBP-1 und ESM-1 eines Individuums mit einer gesunden Schwangerschaft ist; and
(iii) Identifizieren, dass es wahrscheinlich ist, dass das Individuum das schwangerschaftsbedingte Syndrom aufweist oder entwickelt, basierend auf dem Vergleich des Verhältnisses des ersten Parameters und des zweiten Parameters und des Referenzverhältnisses.

2. In-vitro-Verfahren nach Anspruch 1, wobei das Verfahren umfasst:
(ia) Messen der Konzentrationen von GBP-1 und ESM-1 in der biologischen Probe, wobei die biologische Probe vom schwangeren Individuum ist, das sich in einer der Schwangerschaftswochen 10-36 befindet;
(ib) Bestimmen des Verhältnisses zwischen den Konzentrationen von GBP-1 und ESM-1;
(ii) Vergleichen des Verhältnisses mit dem Referenzwert und
(iii) Identifizieren, dass es wahrscheinlich ist, dass das Individuum das schwangerschaftsbedingte Syndrom aufweist oder entwickelt, basierend auf einem Vergleich des Verhältnisses zwischen den Konzentrationen von GBP-1 und ESM-1 und dem Referenzverhältnis.

3. Verfahren nach einem der vorhergehenden Ansprüche, wobei das Bestimmen des Verhältnisses der Konzentrationen von GBP-1 und ESM-1 ein Teilen der GBP-1-Konzentration durch die ESM-1-Konzentration umfasst, wobei
- das Verfahren ferner (iii) ein Identifizieren, dass es wahrscheinlich ist, dass das Individuum, das sich insbesondere in einer der Schwangerschaftswochen 10-16 befindet, das schwangerschaftsbedingte Syndrom entwickelt, basierend auf einem Vergleich des Verhältnisses zwischen den Konzentrationen von GBP-1 und ESM-1 mit dem Referenzwert umfasst, wenn das Verhältnis zwischen den Konzentrationen von GBP-1 und ESM-1 größer als der Referenzwert ist; und/oder
- die biologische Flüssigkeitsprobe von einem schwangeren Individuum ist, das sich in einer der Schwangerschaftswochen zwischen Woche 10-18 befindet, und wobei die biologische Flüssigkeitsprobe eine Plasma-oder Serumprobe umfasst, und wobei beurteilt wird, dass das schwangere Individuum das schwangerschaftsbedingten Syndrom aufweist, wenn das Verhältnis zwischen den Konzentrationen von GBP-1 und ESM-1 im Bereich von 7±3 liegt.

4. Verfahren nach einem der vorhergehenden Ansprüche, wobei das Verhältnis zwischen den Konzentrationen von GBP-1 und ESM-1 der biologischen Flüssigkeitsprobe des schwangeren Individuums darauf hinweist, dass es das schwangerschaftsbedingte Syndrom aufweist oder entwickelt, wenn das Verhältnis 400 % des Verhältnisses zwischen den Konzentrationen von GBP-1 und ESM-1 der biologischen Flüssigkeitsprobe von schwangeren Individuen mit einer gesunden Schwangerschaft entspricht oder darüber liegt.

5. Verfahren nach einem der vorhergehenden Ansprüche, ferner umfassend ein Verwenden eines für GBP-1 oder ESM-1 spezifischen Assays, wobei der Assay einen ELISA-Assay (Enzymimmunassay), einen turbidimetrischen Assay, einen Radioimmunosorbent-Assay (RIST), einen Radioimmunassay (RIA), einen direkten oder indirekten Immunfluoreszenzassay, einen Partikel-Gel-Immunassay (PaGIA) oder einen Lateralfluss-Assay umfasst, wobei der Assay insbesondere einen oder mehrere von einem ELISA, einem Lateralfluss-Assay, einem enzymatischen Assay, einem kolorimetrischen Assay und einem Lumineszenzassay umfasst, und/oder wobei das Messen der Konzentration von ESM-1 in der biologischen Probe ein Verwenden von Massenspektrometrie (MS) umfasst; wobei das Verfahren ferner die Stufe eines Gebärmutterarterien-Doppler-Screenings und Ableitens von Informationen über die Plazentation daraus umfasst.

6. Verfahren nach einem der vorhergehenden Ansprüche, wobei die Konzentration von GBP-1 und ESM-1 der Spiegel von freiem GBP-1 und/oder freiem ESM-1 in der biologischen Probe ist, und/oder wobei die Konzentration von GBP-1 und ESM-1 der Spiegel von GBP-1 und/oder ESM-1 ist, die an ihren jeweiligen Agonisten in der biologischen Probe gebunden oder damit komplexiert sind.

7. Verfahren nach einem der vorhergehenden Ansprüche, wobei eines oder mehreres von Folgendem: (a) das Messen der Konzentration von GBP-1 und/oder ESM-1 in der biologischen Probe umfasst ein Verfahren zum Durchführen einer Quantifizierung von RNA, die für GBP-1 und/oder ESM-1 codiert, in den Zellen oder zellfreier RNA in der biologischen Probe, und wobei (b) das Messen der Konzentration von GBP-1 und/oder ESM-1 in der biologischen Probe ein Verfahren zum Quantifizieren der Proteinkonzentration durch die Konzentration von RNA umfasst, die für ESM-1 codiert; und wobei die Quantifizierung durch RT-PCR, PCR, Hybridisierung oder andere Mittel zum Bestimmen der Konzentration von Nucleotiden erfolgt.

8. Verfahren nach einem der vorhergehenden Ansprüche, wobei das Verfahren ein Bereitstellen einer ersten biologischen Probe von einem Individuum, die bei einer ersten Gelegenheit entnommen wurde, und Bereitstellen einer zweiten biologischen Probe vom Individuum, die bei einer zweiten Gelegenheit entnommen wurde, Messen der Konzentration von GBP-1 und ESM-1 in der ersten biologischen Probe, die eine erste Konzentration darstellt, Verwenden eines für GBP-1 und ESM-1 spezifischen Assays und Messen der Konzentration von GBP-1 und ESM-1 in der zweiten biologischen Probe umfasst, die eine zweite Konzentration darstellt, und wobei das Verfahren ferner ein Identifizieren, dass es wahrscheinlich ist, dass das Individuum das schwangerschaftsbedingte Syndrom aufweist oder entwickelt, basierend auf einem Vergleich des ersten Verhältnisses zwischen den zwei Markern aus den ersten Konzentrationen und des Verhältnisses zwischen den zwei Markern aus den zweiten Konzentrationen umfasst.

9. Vorrichtung zum Identifizieren eines schwangerschaftsbedingten Syndroms, das aus der Gruppe bestehend aus Präeklampsie, Eklampsie, und Hämolyse erhöhten Leberenzymen und niedriger Thrombozytenzahl (HELLP) ausgewählt ist, aus einer biologischen Flüssigkeitsprobe von einem schwangeren Individuum, wobei die biologische Flüssigkeitsprobe eine Blutprobe, eine Plasmaprobe oder eine Serumprobe vom schwangeren Individuum umfasst, wobei die Vorrichtung (a) eine Analyseeinheit, die so konfiguriert ist, dass sie einen ersten Parameter, der mit der Konzentration von GBP-1 in der biologischen Flüssigkeitsprobe skaliert, und einen zweiten Parameter bestimmt, der mit der Konzentration von ESM-1 in der biologischen Flüssigkeitsprobe skaliert, und ein Verhältnis des ersten Parameters und des zweiten Parameters definiert; und (b) eine Auswerteeinheit umfasst, die einen Datenprozessor umfasst, der erforderliche Algorithmen zum Bestimmen eines Verhältnisses zwischen dem ersten Parameter und dem zweiten Parameter und Vergleichen des Verhältnisses mit einem in einer Datenbank gespeicherten Referenzwert implementiert aufweist, um zu bestimmen, ob es wahrscheinlich ist, dass ein schwangeres Individuum Präeklampsie, Eklampsie und/oder HELLP aufweist oder entwickelt, wobei der Referenzwert ein Referenzverhältnis von GBP-1 und ESM-1 eines Individuums mit einer gesunden Schwangerschaft ist.

10. Vorrichtung nach Anspruch 9 zum Identifizieren, dass es wahrscheinlich ist, dass ein schwangeres Individuum, das sich in einer der Schwangerschaftswochen 10-36 befindet, ein schwangerschaftsbedingtes Syndrom aufweist oder entwickelt, das aus der Gruppe bestehend aus Präeklampsie, Eklampsie und HELLP ausgewählt ist, wobei die Vorrichtung (a) eine Analyseeinheit mit Nachweismitteln für GBP-1 und ESM-1, wobei es sich zum Beispiel um eine Kombination von Antikörpern, die für GBP-1 und/oder ESM-1 spezifisch sind, einen (rekombinanten) Rezeptor, der für GBP-1 und/oder ESM-1 spezifisch ist, oder ein Aptamer handelt, das für GBP-1 und/oder ESM-1 spezifisch ist, welche die Bestimmung der Konzentration von GBP-1 und ESM-1 ermöglicht; und (b) eine Auswerteeinheit umfasst, die einen Datenprozessor umfasst, der erforderliche Algorithmen zum Bestimmen der einzelnen Konzentrationen und des Verhältnisses zwischen den Konzentrationen und Vergleichen des Verhältnisses mit einem in einer Datenbank gespeicherten Referenzwert implementiert aufweist, um zu bestimmen, ob es wahrscheinlich ist, dass ein schwangeres Individuum Präeklampsie, Eklampsie und/oder HELLP aufweist oder entwickelt.

11. Computer-implementierte Verwendung einer Auswertung der Konzentration von GBP-1 und ESM-1 oder ihres Verhältnisses in einer biologischen Flüssigkeitsprobe, die einem schwangeren Individuum entnommen wurde, das sich in einer der Schwangerschaftswochen 10-36, beispielsweise 10-18, befindet, zum Identifizieren, ob es wahrscheinlich ist, dass das schwangere Individuum ein schwangerschaftsbedingtes Syndrom aufweist oder entwickelt, das aus der Gruppe bestehend aus Präeklampsie, Eklampsie und/oder HELLP ausgewählt ist, wobei die biologische Flüssigkeitsprobe eine Blutprobe, eine Plasmaprobe oder eine Serumprobe vom schwangeren Individuum umfasst.

12. In-vitro-Verwendung einer biologischen Flüssigkeitsprobe, die einem schwangeren Individuum entnommen wurde, das sich in einer der Schwangerschaftswochen 10-36 befindet, zum Identifizieren, ob es wahrscheinlich ist, dass das schwangere Individuum ein schwangerschaftsbedingtes Syndrom aufweist oder entwickelt, das aus der Gruppe bestehend aus Präeklampsie, Eklampsie und HELLP ausgewählt ist, wobei die biologische Flüssigkeitsprobe eine Blutprobe, eine Plasmaprobe oder eine Serumprobe vom schwangeren Individuum umfasst, wobei die Verwendung umfasst: (a) Messen der Konzentrationen von GBP-1 und ESM-1 in der biologischen Probe; (b) Vergleichen der Konzentrationen von GBP-1 und ESM-1 oder ihres Verhältnisses mit einem Referenzwert; und (c) Identifizieren, dass es wahrscheinlich ist, dass das Individuum das schwangerschaftsbedingte Syndrom aufweist oder entwickelt.

13. Kit zum Bestimmen eines schwangerschaftsbedingten Syndroms, das aus der Gruppe bestehend aus Präeklampsie, Eklampsie und/oder HELLP ausgewählt ist, aus einer biologischen Flüssigkeitsprobe von einem schwangeren Individuum, wobei die biologische Flüssigkeitsprobe eine Blutprobe, eine Plasmaprobe oder eine Serumprobe vom schwangeren Individuum umfasst, wobei der Kit einen ELISA für GBP-1, einen ELISA für ESM-1 und ein Handbuch umfasst, das Referenzwerte bereitstellt, auf deren Basis bestimmt werden kann, ob es wahrscheinlich ist, dass das schwangere Individuum eines der schwangerschaftsbedingten Syndrome aufweist oder entwickelt.

14. Kit zum Identifizieren, ob es wahrscheinlich ist, dass ein schwangeres Individuum, das sich in einer der Schwangerschaftswochen 10-36 befindet, ein schwangerschaftsbedingtes Syndrom aufweist oder entwickelt, das aus der Gruppe bestehend aus Präeklampsie, Eklampsie und HELLP ausgewählt ist, wobei der Kit (a) eine Analyseeinheit mit einem Nachweismittel für ESM-1 und eine Analyseeinheit mit einem Nachweismittel für GBP-1 sowie ein Nachweismittel für eines oder mehrere von sF1t1, VEGF, P1GF, HGF, sEndoglin, pp-13, PAPP-A, GDF-15, Pikachurin und Hämopexin; und (b) eine Auswerteeinheit zum Bestimmen, ob es wahrscheinlich, dass ein schwangeres Individuum das schwangerschaftsbedingte Syndrom aufweist oder entwickelt, basierend auf einem Ergebnis umfasst, das von der Analyseeinheit bereitgestellt wird, insbesondere umfassend eine Auswerteeinheit, die einen Datenprozessor umfasst, der erforderliche Algorithmen zum Vergleichen der Konzentrationen von GBP-1 und ESM-1 oder ihres Verhältnisses mit einem von sF1t1, VEGF, P1GF, HGF, sEndoglin, pp-13, PAPP-A, GDF-15, Pikachurin und Hämopexin, bestimmt mit in einer Datenbank gespeicherten Referenzwerten, und Berechnen des Verhältnisses zwischen den gemessenen Markern implementiert aufweist, um zu bestimmen, ob es wahrscheinlich ist, dass ein schwangeres Individuum das schwangerschaftsbedingte Syndrom aufweist oder entwickelt.

15. Kit nach Anspruch 14, ferner umfassend ein Handbuch oder einen Verweis auf ein Handbuch, wobei das Handbuch Anleitungen umfasst, wie eine biologische Flüssigkeitsprobe einem schwangeren Individuum zu entnehmen ist, und/oder wie die Analyseeinheit zu verwenden ist, und/oder wie die Auswerteeinheit zu verwenden ist, wobei das Handbuch Informationen zum Entnehmen einer biologischen Flüssigkeitsprobe von einem schwangeren Individuum in einer Schwangerschaftswochen 10-36 umfasst, und wobei die Auswerteeinheit ein Farbschema umfasst, wobei die Analyseeinheit zum Bereitstellen einer Farbreaktion konfiguriert ist, wobei die Farbe vom Verhältnis abhängt, das auf den Konzentrationen von GBP-1 und ESM-1 in einer biologischen Probe basiert, die einem schwangeren Individuum entnommen wurde.

16. Verwendung eines Kits nach einem der Ansprüche 13-15 zum Vorhersagen, dass ein schwangeres Individuum in einer der Schwangerschaftswochen 10-36, beispielsweise 10-18, nach Woche 20 früh einsetzende schwere Präeklampsie entwickelt, basierend auf einer Messung der Konzentrationen von GBP-1 und ESM-1 oder dem Verhältnis zwischen den beiden in einer biologischen Flüssigkeitsprobe vom schwangeren Individuum, wobei die biologische Flüssigkeitsprobe eine Blutprobe, eine Plasmaprobe oder eine Serumprobe vom schwangeren Individuum umfasst.

## Revendications

1. Procédé in vitro permettant d'identifier à partir d'un échantillon de fluide biologique provenant d'une patiente enceinte un syndrome lié à la grossesse choisi dans le groupe constitué par la prééclampsie, l'éclampsie et le syndrome HTFEPB (hémolyse, tests du foie élevés, plaquettes basses), dans lequel l'échantillon de fluide biologique comprend un échantillon de sang, un échantillon de plasma ou un échantillon de sérum provenant de la patiente enceinte, le procédé consistant à
(i) déterminer un premier paramètre qui évolue avec la concentration de GBP-1 dans ledit échantillon de fluide biologique et un second paramètre qui évolue avec la concentration d'ESM-1 dans ledit échantillon de fluide biologique, et définir un rapport du premier paramètre et du second paramètre ;
(ii) comparer ce rapport du premier paramètre et du second paramètre avec une valeur de référence, la valeur de référence étant un rapport de référence de GBP-1 et d'ESM-1 d'une patiente ayant une grossesse saine ; et
(iii) identifier la patiente comme étant susceptible d'avoir ou de développer le syndrome lié à la grossesse sur la base de la comparaison du rapport du premier paramètre et du second paramètre et du rapport de référence.

2. Procédé in vitro selon la revendication 1, le procédé consistant à
(ia) mesurer les concentrations de GBP-1 et d'ESM-1 dans l'échantillon biologique, l'échantillon biologique provenant de la patiente enceinte qui est dans l'une quelconque des semaines 10-36 ;
(ib) déterminer le rapport entre les concentrations de GBP-1 et d'ESM-1 ;
(ii)comparer ledit rapport à la valeur de référence, et
(iii) identifier la patiente comme étant susceptible d'avoir ou de développer le syndrome lié à la grossesse sur la base d'une comparaison du rapport entre les concentrations de GBP-1 et d'ESM-1 avec la valeur de référence.

3. Procédé selon l'une quelconque des revendications précédentes, dans lequel la détermination du rapport entre les concentrations de GBP-1 et d'ESM-1 consiste à diviser la concentration de GBP-1 par la concentration d'ESM-1, dans lequel
- le procédé consistant en outre à (iii) identifier la patiente, en particulier étant dans l'une quelconque des semaines 10-16 de grossesse, comme étant susceptible de développer le syndrome lié à la grossesse sur la base d'une comparaison du rapport entre les concentrations de GBP-1 et d'ESM-1 avec la valeur de référence lorsque ledit rapport entre les concentrations de GBP-1 et d'ESM-1 est supérieur à la valeur de référence ; et/ou
- ledit échantillon de fluide biologique provient d'une patiente enceinte qui se trouve dans une quelconque semaine de grossesse entre les semaines 10 et 18, et dans lequel ledit échantillon de fluide biologique comprend un échantillon de plasma ou de sérum et dans lequel la patiente enceinte est évaluée comme ayant le syndrome lié à la grossesse lorsque le rapport entre les concentrations de GBP-1 et d'ESM-1 se situe dans la plage de 7 ± 3.

4. Procédé selon l'une quelconque des revendications précédentes, dans lequel le rapport entre les concentrations de GBP-1 et d'ESM-1 de l'échantillon de fluide biologique de la patiente enceinte est révélateur du fait qu'elle a ou développe le syndrome lié à la grossesse lorsque le rapport est égal ou supérieur à 400 % du rapport entre les concentrations de GBP-1 et d'ESM-1 des échantillons de fluide biologique des patientes enceintes ayant une grossesse saine.

5. Procédé selon l'une quelconque des revendications précédentes, consistant en outre à utiliser un dosage spécifique à GBP-1 ou ESM-1, dans lequel le dosage comprend un test ELISA (test d'immunoabsorption par enzyme liée), un dosage par turbidimétrie, un radio-immuno-sorbent test (RIST), un dosage radio-immunométrique (RIA), un test d'immunofluorescence directe ou indirecte, un immunodosage sur gel avec particules (PaGIA), ou un dosage par flux latéral, en particulier dans lequel le dosage comprend un ou plusieurs parmi un test ELISA, un dosage par flux latéral, un dosage enzymatique, un dosage colorimétrique et un dosage par luminescence, et/ou dans lequel la mesure de la concentration d'ESM-1 dans l'échantillon biologique consiste à utiliser une spectrométrie de masse (SM) ; le procédé comportant en outre l'étape consistant à réaliser un dépistage Doppler de l'artère utérine et à dériver de celui-ci des informations concernant la placentation.

6. Procédé selon l'une quelconque des revendications précédentes, dans lequel ladite concentration de GBP-1 et d'ESM-1 est le niveau de GBP-1 libre et/ou d'ESM-1 libre dans l'échantillon biologique, et/ou dans lequel ladite concentration de GBP-1 et d'ESM-1 est le niveau de GBP-1 et/ou d'ESM-1 liés à leur agoniste respectif, ou complexés avec celui-ci, dans l'échantillon biologique.

7. Procédé selon l'une quelconque des revendications précédentes, dans lequel (a) la mesure de la concentration de GBP-1 et/ou d'ESM-1 dans l'échantillon biologique comprend un procédé pour réaliser une quantification d'ARN codant pour GBP-1 et/ou ESM-1 dans des cellules ou un ARN acellulaire dans l'échantillon biologique, et/ou dans lequel (b) la mesure de la concentration de GBP-1 et/ou d'ESM-1 dans l'échantillon biologique comprend un procédé pour quantifier la concentration de protéine par l'intermédiaire de la concentration d'ARN codant pour ESM-1 ; et dans lequel ladite quantification est réalisée par le biais d'une RT-PCR, d'une PCR, d'une hybridation ou d'autres moyens pour déterminer la concentration de nucléotides.

8. Procédé selon l'une quelconque des revendications précédentes, le procédé consistant à fournir un premier échantillon biologique provenant d'une patiente prélevé lors d'une première occasion, et à fournir un second échantillon biologique provenant de la patiente prélevé lors d'une seconde occasion, à mesurer la concentration de GBP-1 et d'ESM-1 dans le premier échantillon biologique, à constituer une première concentration, à utiliser un dosage spécifique à GBP-1 et ESM-1, à mesurer la concentration de GBP-1 et d'ESM-1 dans le second échantillon biologique, et à constituer une seconde concentration, et le procédé consistant en outre à identifier la patiente comme étant susceptible d'avoir ou de développer le syndrome lié à la grossesse sur la base d'une comparaison du premier rapport entre les deux marqueurs provenant des premières concentrations et du rapport entre les deux marqueurs provenant des secondes concentrations.

9. Dispositif permettant d'identifier à partir d'un échantillon de fluide biologique provenant d'une patiente enceinte un syndrome lié à la grossesse choisi dans le groupe constitué par la prééclampsie, l'éclampsie et le syndrome HTFEPB (hémolyse, tests du foie élevés, plaquettes basses), dans lequel l'échantillon de fluide biologique comprend un échantillon de sang, un échantillon de plasma ou un échantillon de sérum provenant de la patiente enceinte, ledit dispositif comprenant (a) une unité d'analyse configurée pour déterminer un premier paramètre qui évolue avec la concentration de GBP-1 dans ledit échantillon de fluide biologique et un second paramètre qui évolue avec la concentration de ESM-1 dans ledit échantillon de fluide biologique ; (b) une unité d'évaluation comprenant un processeur de données ayant implémenté des algorithmes nécessaires pour déterminer un rapport entre le premier paramètre et le second paramètre et pour comparer le rapport à une valeur de référence mémorisée dans une base de données dans le but de déterminer si une patiente enceinte est susceptible d'avoir ou de développer une prééclampsie, une éclampsie et/ou un syndrome HTFEPB, dans lequel la valeur de référence est un rapport de référence de GBP-1 et d'ESM-1 d'une patiente ayant une grossesse saine.

10. Dispositif selon la revendication 9, permettant d'identifier une patiente enceinte lorsqu'elle est dans l'une des semaines 10-36 de grossesse comme étant susceptible d'avoir ou de développer un syndrome lié à la grossesse choisi dans le groupe constitué par la prééclampsie, l'éclampsie et le syndrome HTFEPB, ledit dispositif comprenant (a) une unité d'analyse comprenant des agents de détection pour GBP-1 et ESM-1, par exemple étant une association d'anticorps spécifiques pour GBP-1 et/ou ESM-1, un récepteur (recombinant) spécifique pour GBP-1 et/ou ESM-1 ou un aptamère spécifique pour GBP-1 et/ou ESM-1, ce qui permet la détermination des concentrations de GBP-1 et d'ESM-1 ; (b) une unité d'évaluation comprenant un processeur de données ayant implémenté des algorithmes nécessaires pour déterminer les concentrations individuelles, le rapport entre les concentrations et comparer le rapport à une valeur de référence mémorisée dans une base de données dans le but de déterminer si une patiente enceinte est susceptible d'avoir ou de développer une prééclampsie, une éclampsie et/ou un syndrome HTFEPB.

11. Utilisation implémentée par ordinateur d'une évaluation de la concentration de GBP-1 et d'ESM-1 ou de leur rapport dans un échantillon de fluide biologique prélevé sur une patiente enceinte étant dans l'une quelconque des semaines 10-36, notamment les semaines 10-18 de grossesse, pour identifier si la patiente enceinte est susceptible d'avoir ou de développer un syndrome lié à la grossesse choisi dans le groupe constitué par la prééclampsie, l'éclampsie et le syndrome HTFEPB, dans laquelle l'échantillon de fluide biologique comprend un échantillon de sang, un échantillon de plasma ou un échantillon de sérum provenant de la patiente enceinte.

12. Utilisation in vitro d'un échantillon de fluide biologique prélevé sur une patiente enceinte étant dans l'une quelconque des semaines 10-36 de grossesse pour identifier si la patiente enceinte est susceptible d'avoir ou de développer un syndrome lié à la grossesse choisi dans le groupe constitué par la prééclampsie, l'éclampsie et le syndrome HTFEPB, dans laquelle l'échantillon de fluide biologique comprend un échantillon de sang, un échantillon de plasma ou un échantillon de sérum provenant de la patiente enceinte, l'utilisation consistant à : (a) mesurer les concentrations de GBP-1 et d'ESM-1 dans l'échantillon biologique ; (b) comparer lesdites concentrations de GBP-1 et d'ESM-1 ou leur rapport avec une valeur de référence ; et (c) identifier la patiente comme étant susceptible d'avoir ou de développer le syndrome lié à la grossesse.

13. Kit pour déterminer à partir d'un échantillon de fluide biologique provenant d'une patiente enceinte un syndrome lié à la grossesse choisi dans le groupe constitué par la prééclampsie, l'éclampsie et le syndrome HTFEPB, dans lequel l'échantillon de fluide biologique comprend un échantillon de sang, un échantillon de plasma ou un échantillon de sérum provenant de la patiente enceinte, le kit comprenant un test ELISA pour GBP-1, un test ELISA pour ESM-1, et un manuel ou une référence à un manuel fournissant des valeurs de référence sur la base desquelles il est possible de déterminer si la patiente enceinte a ou est susceptible de développer un des syndromes liés à la grossesse.

14. Kit pour identifier si une patiente enceinte qui est dans l'une quelconque des semaines 10-36 de grossesse est susceptible d'avoir ou de développer un syndrome lié à la grossesse choisi dans le groupe constitué par la prééclampsie, l'éclampsie et le syndrome HTFEPB, ledit kit comprenant : (a) une unité d'analyse comprenant un agent de détection pour ESM-1 et une unité d'analyse comprenant un agent de détection pour GBP-1 ; et un agent de détection pour un ou plusieurs parmi sFlt1, VEGF, P1GF, HGF, s-endogline, pp-13, PAPP-A, GDF-15, pikachurine et hémopexine ; (b) une unité d'évaluation pour déterminer si une patiente enceinte est susceptible d'avoir ou de développer le syndrome lié à la grossesse, sur la base d'un résultat fourni par l'unité d'analyse, en particulier comprenant une unité d'évaluation comprenant un processeur de données ayant implémenté des algorithmes nécessaires pour comparer les concentrations de GBP-1 et d'ESM-1, ou leur rapport, à un ou plusieurs parmi sFlt1, VEGF, P1GF, HGF, s-endogline, pp-13, PAPP-A, GDF-15, pikachurine et hémopexine déterminés avec des valeurs de référence mémorisées dans une base de données et pour calculer le rapport entre les marqueurs mesurés dans le but de déterminer si une patiente enceinte est susceptible d'avoir ou de développer le syndrome lié à la grossesse.

15. Kit selon la revendication 14, comprenant en outre un manuel ou une référence à un manuel, dans lequel le manuel comprend des instructions sur la façon de prélever un échantillon de fluide biologique sur une patiente enceinte et/ou sur la façon d'utiliser l'unité d'analyse et/ou sur la façon d'utiliser l'unité d'évaluation, dans lequel le manuel comprend des informations pour prélever l'échantillon de fluide biologique sur une patiente enceinte qui est dans l'une quelconque des semaines 10-36 de grossesse, et dans lequel l'unité d'évaluation comprend un schéma de couleurs, dans lequel l'unité d'analyse est configurée pour fournir une réaction colorée dans laquelle la couleur dépend du rapport basé sur les concentrations de GBP-1 et d'ESM-1 dans un échantillon biologique prélevé sur une patiente enceinte.

16. Utilisation d'un kit selon l'une quelconque des revendications 13-15, permettant de prédire qu'une patiente enceinte dans une quelconque des semaines 10-36, comme les semaines 10-18 de grossesse, développe après la semaine 20 une prééclampsie sévère à début précoce, sur la base d'une mesure des concentrations de GBP-1 et d'ESM-1 ou du rapport entre les deux dans un échantillon de fluide biologique provenant de la patiente enceinte, dans lequel l'échantillon de fluide biologique comprend un échantillon de sang, un échantillon de plasma ou un échantillon de sérum provenant de ladite patiente enceinte.
